Europäisches Patentamt

European Patent Office

Office européen des brevets

(11) Publication number: **0 212 866 B1**

## EUROPEAN PATENT SPECIFICATION

(45) Date of publication of patent specification: **19.08.92**    (51) Int. Cl.⁵: **A01N  33/04**

(21) Application number: **86305739.4**

(22) Date of filing: **25.07.86**

(54) **Nonirritating teat dip.**

(30) Priority: **29.07.85 US 760241**

(43) Date of publication of application:
**04.03.87 Bulletin  87/10**

(45) Publication of the grant of the patent:
**19.08.92 Bulletin  92/34**

(84) Designated Contracting States:
**DE FR GB IT NL**

(56) References cited:
**US-A- 2 246 524**

**COMMONWEALTH AGRICULTURAL BU-REAUX, no. 73592353, Slough, GB; D. SIPKA: "Microbiological studies of the efficacy of disinfectants used in dairies", & ARCHIV FÜR LEBENSMITTELHYGIENE, vol. 23, no. 8, 1972, p. 176-179 (Cat.D)**

**COMMONWEALTH AGRICULTURAL BU-REAUX, no. 76894029, Slough, GB; M. TEOFANOVIC: "Value of disinfection in pre-vention of causative factors of mastitis on machine-milking farms", & VETERINARSKI GLASNIK, vol. 28, no. 10, 1974, p. 781-786**

(73) Proprietor: **MINNESOTA MINING AND MANU-FACTURING COMPANY
3M Center, P.O. Box 33427
St. Paul, Minnesota 55133-3427(US)**

(72) Inventor: **Andrews, Jeffrey F. c/o Minnesota Mining and
Manufacturing Co. 2501 Hudson Road P.O. Box 33427
St. Paul Minnesota 55133-3427(US)**

(74) Representative: **Baillie, Iain Cameron et al c/o Ladas & Parry, Altheimer Eck 2
W-8000 München 2(DE)**

## Description

Technical Field

This invention relates to a novel teat dip comprising a germicidal agent. This invention also relates to a method for controlling mastitis in dairy cows using such a teat dip.

Background of the Invention

The formulation of a teat dip is a complex process which requires that numerous ingredients be compatible and provide an effective product. Barrier-type teat dips and germicidal teat dips and combinations of the two are the main types of dips known. In practice, a teat is dipped into a teat dip subsequent to milking and the resulting film is allowed to remain on the teat until the next milking which is generally 8 to 12 hours later.

Although many germicides have been tried in teat dips, it is very difficult to find germicides which are satisfactory. A satisfactory germicide must be sufficiently effective in killing various microbes, provide a stable dip formulation, be nonirritating to the teat for the period during which it remains on the teat, and be relatively economical, since it is the major active ingredient in the formulation.

Some of the prior art germicides described as useful in teat dips include chlorinated cyanurates (U.S. Patent 4,025,628), chlorhexidine (U.S. Patent 4,434,181), alkyl quaternary ammonium salts (U.S. Patent 3,993,777), iodine (U.S. Patent 2,739,922) and combinations of nitroalkanols and aminocarboxylic acids (U.S. Patent 4,199,602).

"Tego 51" and "Tego 51B" are germicidal products available from Th. Goldschmidt A.G. (Essen, West Germany). These products are complex mixtures of a variety of alkylamines and N-substituted glycines. Analysis conducted on behalf of the inventor has indicated that the major germicidal components present are 3-(n-dodecylamino)propylamine and 2-[2-(n-dodecylamino)ethylamino]ethylamine.

A standard primary skin irritation test on rabbits (Draize test) run on "Tego 51" containing 9% "active ingredients" showed the product to be severly corrosive to skin. A severe erythema with eschar formation and severe edema was followed within seven (7) days by complete necrosis of the skin at the test site. Even when diluted to 1.0% and 0.5% concentrations, the "Tego 51" showed moderate to severe erythema and edema after 24 hours. Moderate necrosis was also seen at these concentrations. The above data are included in Report No. R-2846 (dated March 1969) provided by Th. Goldschmidt A.G., the manufacturer of "Tego 51".

M. Sipka, Arch. Lebensmittelhygiene, 1972, 23(8), 176-9 discloses the use of "Tego 51/15 DL" as an udder wash. Similarly, vendor literature provided by Th. Goldschmidt A.G. discloses the use of "Tego 51" as an udder wash. Use of a product as an udder wash involves contact of the product with the teat for only a short period of time on the order of seconds or at most a minute or two just prior to milking.

Germicides as obviously irritating as "Tego 51" and "Tego 51B" would not usually be considered suitable for use in a teat dip which entails prolonged contact of the germicide with the teat, even though their potency, spectrum of activity and cost make them otherwise very attractive candidates. Even more unlikely would be their use in a concentrated teat dip because of the chance of severe injury to the cow if the product was accidentally used undiluted. Surprisingly, however, a way to use these potent germicides in a teat dip suitable for daily use has been found.

USP 2,246,524 discloses a class of N-alkylated alkylene polamines and their salts for use as germicides, antiseptics, bactericides etc. M.S. Balsam et. al. "Cosmetics Science and Technology" (1979) refers to the use of various compounds as emollients and film forming agents in pharmaceutical formulations applied to the skin. The reference does not however teach an inventive selection of poly (N-vinylpyrrolidone) as a film forming agent and a suitable emollient said selected compounds being compatible with each other and the germicide composition which together form a substantially stable homogeneous non-irritating teat dip mixture.

## Summary of the Invention

The present invention relates to improved animal teat dip formulations. In particular, it relates to novel teat dips which are effective and yet non-irritating even when applied as a concentrate. In another embodiment the invention also relates to use of a germicide for preparation of a medicament for controlling bovine mastitis in which the medicament is prepared by forming the teat dips of the subject patent.

The teat dips of the present invention are formulations comprising, as a homogeneous mixture, a germicide selected from 3-(n-dodecylamino)-propylamine, a pharmaceutically acceptable acid-addition salt of 3-(n-dodecylamino)propylamine, 2-[2-(n-dodecylamino) ethylamino]ethylamine, and a pharmaceutically acceptable acid-addition salt of 2-[2-(n-dodecylamino)ethylamino]-ethylamine, said germicide or germicides being present in a total amount of 0.25 to 2.5 percent based on the total weight of said teat dip concentrate; as an emollient a polyethoxylated or polypropoxylated glucoside; as a film-forming ingredient poly(N-vinylpyrrolidone); and water. Optionally (and preferably) the formulations further include a dye, preferably a dye generally recognized as safe, and/or an antifoaming agent. Teat dip concentrates are also described.

The formulations of the present invention are provided as concentrates to be diluted with water or as solutions ready to be used on the teats of a cow. Teat dips are ordinarily used and provided as dilute solutions. It is somewhat easier to formulate many of the known dips as dilute mixtures since dilute mixtures are generally more stable than concentrated mixtures. On the other hand, concentrates are much less expensive to provide and to ship. Although concentrates are more difficult to formulate, this invention provides concentrated formulations with excellent stability and properties. In fact, the preferred concentrates of the invention are generally more stable than the corresponding diluted formulations.

It was surprising to find that this invention provided nonirritating concentrated formulations in view of the known skin irritancy of "Tego 51" and "Tego 51B".

Detailed Description of the Invention

The present invention relates to teat dip concentrates and dilute formulations for use in providing germicidal protection to the teats of dairy cows.

As used in the instant specification and claims, "substantially stable" as used in connection with "homogeneous mixture" denotes a homogeneous mixture which remains homogeneous for a period of at least about ninety (90) days at room temperature. By "substantially nonirritating" is meant that teats dipped with teat dip twice daily for twenty one (21) consecutive days show no substantial irritation. By "pharmaceutically acceptable acid addition salt" is meant any salt which exhibits suitable germicidal activity and is not substantially less safe than the free base.

The 3-(n-dodecylamino)propylamine and 2-[2-(n-dodecylamino)ethylamino]ethylamine useful as the germicides in the formulations of the present invention are available commercially in "Tego 51" and "Tego 51B" from Th. Goldschmidt Co., Essen, West Germany.

Both "Tego 51" and "Tego 51B" are aqueous solutions which comprise 3-(n-dodecylamino)propylamine and 2-[2-(n-dodecylamino)ethylamino]ethylamine as the principal germicidal components. It is believed that the principal components are present in "Tego 51" and "Tego 51B" as the hydrochloride salts. Also present in a significant amount is $CH_3(CH_2)_{11}NHCH_2CH_2CH_2NHCH_2COOH$, which may be named N-[3-(n-dodecylamino)propyl]glycine and which too is believed to be present as the hydrochloride salt. The latter compound, however, has been found to be much less active than the former two against Staphylococcus aureus and Escherichia coli. Numerous other components are also present in "Tego 51" and "Tego 51B", but the germicidal activity exhibited by the above "Tego" products is believed to be attributable primarily to those components mentioned above (particularly the first two mentioned).

The teat dip concentrates of the present invention comprising, as a substantially stable homogeneous mixture, a germicide selected from 3-(n-dodecylamino)-propylamine, a pharmaceutically acceptable acid-addition salt of 3-(n-dodecylamino)propylamine, 2-[2-(n-dodecylamino) ethylamino]ethylamine, and a pharmaceutically acceptable acid-addition salt of 2-[2-(n-dodecylamino)ethylamino]-ethylamine, said germicide or germicides being present in a total amount of 0.25 to 2.5 percent based on the total weight of said teat dip, concentrate; as an emollient a polyethoxylated or polypropoxylated glucoside; as a film-forming ingredient poly(N-vinylpyrrolidone); and water. All ranges of amounts indicated in the instant specification and claims for the germicidal component(s) are amounts by weight based on the free base whether or not the free base or a pharmaceutically acceptable salt thereof is employed.

Preferably, the teat dip concentrate of the present invention comprises 3-(n-dodecylamino)propylamine or the hydrochloride salt thereof in an amount of about 0.25 to 1.8 % by weight, and more preferably about 0.4 to 0.9 % based on the total weight of the concentrate, and further comprises 2-[2-(n-dodecylamino)-ethylamino]ethylamine or the hydrochloride salt thereof in an amount of about 0.05 to 0.7% by weight and more preferably 0.09 to 0.4% based on the total weight of the concentrate.

An ingredient that is preferably used in many commercial teat dips, and is present in the teat dips of the present invention, is an emollient. Most germicides are at least mildly irritating to skin and an ingredient to counteract any potential irritation is essential. It is well known to researchers and dairymen that irritated teats are more prone to mastitis infection than are soft, healthy teats. Several emollient, anti-irritant, or moisturizing type ingredients were evaluated. Water soluble, acetylated polyethoxylated lanolin derivatives such as Amerchol's "Solulan 97" and "Solulan 98" were tried unsuccessfully since a stable homogeneous mixture did not result. These gave cloudy solutions which separated and gelled after only 2 days aging. Glycerine was tried but was also incompatible with the germicide ("Tego 51B") since a stable homogeneous mixture did not result. A ring of gelled material formed at the top of the solution when these two were combined. The same phenomenon occurred when propylene glycol was tested as an emollient with "Tego 51B" germicide.

Suitable emollients are the polyethylene glycol methyl glucosides such as "Glucamate DOE 120" (a polyethoxylated glucose dioleate containing 120 ethoxy units in the polyethylene glycol moiety, available from Amerchol Corporation, Edison, N.J.); "Glucam E10" (a polyethoxylated methyl glucose containing 10 ethoxy units from Amerchol Corporation); "Glucam E-20" (a polyethoxylated methyl glucose containing 20 ethoxy units in the polyethylene glycol moiety, available from Amerchol Corporation; "Glucam P-10" (a polypropoxylated methyl glucose containing 10 propoxy units in the polyethyleneglycol moiety, available from Amerchol Corporation); and "Glucam P-20" (a polypropoxylated methyl glucose containing 20 propoxy units in the polyethyleneglycol moiety, available from Amerchol Corporation). These emollients provide formulations which would be found to be stable over a range of temperatures from 0° to 50°C. It is also believed that other polyethoxylated glucosides would also be suitable. Other emollients which are suitable are hydrogenated starch hydrolysates available from the trade designations "Hystar C.G.", "Hystar HM-75" and "Hystar TPF", all of which being available from Lonza, Inc., Fairlawn, N. J. Sorbitol would also be suitable.

The emollient should be present in the instant teat dip concentrates in an amount of about 1 to 40%, and preferably 2 to 6% by weight based on the total weight of the concentrate.

A film-forming ingredient is also required in the formulations of the present invention. Several were tried, including xanthan gum, carboxymethylcellulose, poly(vinyl alcohol), hydroxyethyl cellulose and poly-(N-vinylpyrrolidone) (PVP). Surprisingly, only PVP made a stable, homogeneous solution with the "Tego 51B" germicide. The film-forming ingredient is generally employed in an amount of about 0.5 to 8% by weight based on the total weight of the teat dip concentrate. At the lower end of the above range, higher molecular weight film-forming ingredients are preferably used to give sufficient adherence of the diluted formulations to the teats.

A dye is desirably included in the teat dip formulations of the invention. Color on the teat tells farmers that they did indeed dip a particular cow. To preclude any problems with possible contamination of milk, it is preferred that only FDC Certified (food grade) dyes be used. There are 7 FDC dyes available which are generally recognized as safe, and since one of these, FDC Red #3, is now under suspicion of being a carcinogen, only 6 dyes are now considered suitable. FDC Yellow #5 is too light in color to show up well on the teats, and, therefore, it is less suitable. FDC Blue #2 is unstable in the presence of light and, therefore, is less desirable. The remaining colors are FDC Red #4, FDC Yellow #6, FDC Green #3 and FDC Blue #1. All of these colors were evaluated for inclusion in the dip. All of the colors had some slight adverse effect on the bacterial kill-rate of the dip. FDC Blue #1, however, had the least effect (See Example 6 below), and it is presently preferred. Also it is contemplated that certain D and C colors may be suitable for inclusion in the dip.

Many germicidal agents have high foaming characteristics. The germicides present in "Tego 51" and "Tego 51B" may result in foaming, and, for that reason, it is preferred to use an antifoaming agent in the formulations of the present invention. Suitable antifoaming agents are the polymers of dimethylsiloxane which are available from Dow Corning Company, an example of such being "Antifoam C". An antifoaming agent, when employed, will generally be employed in an amount of about 0.05 to 0.5% by weight based on the total weight of the teat dip concentrate.

Some known teat dips include ethylenediaminetetraacetic acid which can act as a chelating agent to remove metal ions from hard water. It has been found that ethylenediaminetetraacetic acid reduces stability and bactericidal effect of the teat dips of the invention, and, for that reason, it is preferred that the teat dips of the present invention be free of ethylenediaminetetraacetic acid.

In practice, the teat dip formulation which is prepared from the teat dip concentrate and used to dip cow teats contains at least about 90% water and preferably at least about 95% water by weight based on the total weight of the diluted formulation. The teat dip formulation should contain a germicidally effective amount of germicide selected from the group consisting of 3-(n-dodecylamino)propylamine and 2-[2-(n-dodecylamino)ethylamino]ethylamine, either as the free bases or acid-addition salts. It is understood that the teat dip may comprise both 3-(n-dodecylamino)propylamine and 2-[2-(n-dodecylamino)ethylamino]-ethylamine (and/or pharmaceutically acceptable acid-addition salts of one or both of the foregoing) in amounts that together amount to a germicidally effective amount. By "germicidally effective amount" as used in the instant specification and claims is meant an amount of germicide that will provide at least about a 4 log reduction of bacterial growth when the method of Example 6 is followed and the exposure time of the inoculum to the dip is two minutes and no organic load is employed. Preferably, the teat dip will comprise both 3-(n-dodecylamino)propylamine and 2-[2-(n-dodecylamino)ethylamino]ethylamine, (or the acid-addition salts of either or both) in amounts by weight at about 0.025 to 0.25% and 0.005 to 0.10%, respectively, both based on the total weight of the teat dip formulation.

The following examples are provided to illustrate the invention, but are not intended to be limiting thereof.

**Example 1**

To 317.5 kg (700 lb) of stirred deionized water was added 0.567 kg (1.25 lb) of the poly-(dimethylsiloxane) formulation commercially available as "Antifoam C" from Dow-Corning Company, Midland, Michigan. After 5 minutes of stirring, the solution was heated to 56 to 63°C (135 to 145°F). During the heating period, 3.78 kg (8.34 lb) of poly(N-vinylpyrrolidone) of molecular weight 30,000 (commercially available under the trade designation "PVP K-30" from GAF Chemical Company, New York, NY) was added slowly, followed by slow addition of 15.12 kg (33.34 lb) of a methyl glucose dioleate derivative of polyethylene glycol containing about 120 units of ethylene glycol per molecule (commercially available under the trade designation "Glucamate DOE 120" from Amerchol Chemical Company, Edison, NJ). Mixing was continued for 15 to 30 minutes until a solution was obtained. To this solution was added 45.40 kg (100.08 lb) of "Tego 51B" obtained from Th. Goldschmidt Co., Essen, West Germany. Heating and mixing were continued at 57 to 63°C (135 to 145°F) for 15 minutes. To this solution was added 0.567 kg (1.25 lb) of FDC Blue #1 (available from Warner-Jenkinson Company, St. Louis, MO), and mixing was continued for 30 minutes. The solution was then allowed to cool to ambient temperature to provide a stable, foam-free concentrate.

The amounts of each ingredient present in the final formulation were:

| | |
|---|---|
| "Tego 51B" | 12.00% |
| "Glucamate DOE 120" | 4.00% |
| "PVP K-30" | 1.00% |
| FDC Blue No. 1 | 0.15% |
| "Antifoam C" | 0.15% |
| Deionized Water | 82.70% |

Example 2

The formulation of Example 1 was diluted with seven volumes of water to provide a solution containing 1.5% by weight of the "Tego 51B". A field trial was run on 120 dairy cows, using National Mastitis Council Protocol B.

Briefly, the National Mastitis Council Protocol B requires that each of the four teats of a cow first be dipped for 1/3 of its length in an inoculum of a given bacterium and then be drained for 5 seconds. Two teats are then dipped full length in the teat dip (over the bacteria treated teats), and the remaining two teats are maintained as untreated controls and are not dipped in the teat dip. This procedure is carried out twice a day (after each milking). Three times per week, milk is taken from each teat, a sample is plated out on suitable growth media and evaluated for bacterial growth.

The cows treated with the diluted dip of this example showed 81.2% less mastitis caused by Staphylococcus aureus and 67.5% less mastitis caused by Streptococcus agalactiae than the untreated controls. This shows that the teat dip is effective according to this nationally recognized protocol.

Example 3

A teat dip concentrate formulation was prepared using the method of Example 1 except the amount of "Tego 51B" used was reduced by 50% and the amount of water correspondingly increased.

The amounts of each ingredient in the final formulation were:

| "Tego 51B" | 6.00% |
|---|---|
| "Glucamate DOE 120" | 4.00% |
| "PVP K-30" | 1.00% |
| FDC Blue No. 1 | 0.15% |
| "Antifoam C" | 0.15% |
| Deionized water | 88.70% |

Example 4

The formulation of Example 3 was diluted with seven volumes of water to provide a solution containing 0.75% by weight of the "Tego 51B". A field trial was run as described in Example 2, this time on 40 dairy cows. The cows treated with the dip of Example 3 showed 52.4% less mastitis caused by Staphylococcus aureus but no reduction of mastitis caused by Streptococcus agalactiae compared to the untreated controls. This was considered a successful reduction of mastitis caused by Staphylococcus organisms.

Example 5

The teat dip concentrate formulation of Example 1 (i.e., the formulation containing 12.00% of the "Tego 51B") was applied to all of the teats of four cows in a field trial using normal procedures (i.e., applied twice a day, after each milking) and permitted to dry. It was observed that none of the cows showed irritation of their teats after 21 days since there was no scabbing, chapping or redness.

Example 6

Teat dip formulations were prepared according to the procedure of Example 1 using the several different dyes indicated in Table I below. The bacterial kill rate of these formulations diluted with seven parts of water was measured versus two organisms, Staphylococcus aureus and Escherichia coli using two different exposure times and with and without an organic load (i.e., 10% milk added to the formulations). The test was run by i) adding a 0.1 ml inoculum containing about $10^8$ bacteria per ml to 20 ml of the dip formulation, ii) withdrawing a 0.1 ml aliquot from said formulation and placing it on an agar plate, and iii) incubating the agar plate for 48 hours. The results were compared to untreated controls. The results are shown in Table I. The temperature of the test was 30°C., and the kill rates are shown as reductions of the total number of bacteria present on a log scale.

6

TABLE I

| Dye | NO ORGANIC LOAD | | 10% ORGANIC LOAD | |
|---|---|---|---|---|
| | EXPOSURE TIME | | | |
| | 2 Min. | 10 Min. | 2 Min. | 10 Min. |
| FDC Blue #1 | | | | |
| Staph. aureus | >6.15 | >6.15 | 2.30 | 5.01 |
| E. coli | >5.90 | >5.90 | 4.33 | >5.90 |
| FDC Green #3 | | | | |
| Staph. aureus | 3.79 | 5.01 | 2.85 | 5.53 |
| E. coli | >5.90 | >5.90 | 4.80 | >5.90 |
| FDC Red #4 | | | | |
| Staph. aureus | >6.15 | >6.15 | 1.99 | 4.35 |
| E. coli | >5.90 | >5.90 | 2.04 | >5.90 |
| FDC Yellow #6 | | | | |
| Staph. aureus | >6.15 | >6.15 | TNTC* | 3.85 |
| E. coli | >5.90 | >5.90 | 3.06 | >5.90 |

\* TNTC = too numerous to count

**Claims**

1.  A germicidal animal teat dip concentrate comprising, as a substantially stable homogeneous mixture, a germicide selected from 3-(n-dodecylamino)propylamine, a pharmaceutically acceptable acid-addition salt of 3-(n-dodecylamino)propylamine, 2-[2-(n-dodecylamino) ethylamino]ethylamine, and a pharmaceutically acceptable acid-addition salt of 2-[2-(n-dodecylamino)ethylamino]ethylamine, said germicide or germicides being present in a total amount of 0.25 to 2.5 percent based on the total weight of said teat dip concentrate; as an emollient a polyethoxylated or polypropoxylated glucoside; as a film-forming ingredient poly(N-vinylpyrrolidone); and water.

2.  A teat dip concentrate according to claim 1, further comprising N-[3-n- dodecylamino)propyl]glycine or a pharmaceutically acceptable acid-addition salt thereof.

3.  A teat dip concentrate according to either of claims 1 and 2, wherein said dip concentrate comprises 3-(n-dodecylamino)propylamine in an amount of 0.2 to 1.8 percent based on the total weight of said teat dip concentrate, and 2-[2-(n-dodecylamino) ethylamino] ethylamine or the hydrochloride salt thereof in an amount of 0.05 to 0.7 percent based on the total weight of said teat dip concentrate.

4.  A teat dip from a concentrate concentrate according to claim 1 comprising a germicidally effective amount of said germicide, water being present in an amount of at least about 90% by weight based on the total weight of said teat dip.

5.  A teat dip according to claim 4, further comprising N-[3-(n-dodecylamino)propyl]glycine or a phar-

7

maceutically acceptable acid-addition salt thereof.

6.  A teat dip according to either of claims 4 and 5, wherein said teat dip comprises 3-(n-dodecylamino)-propylamine or the hydrochloride salt thereof in an amount of 0.025 to 0.25 percent based on the total weight of said teat dip; and 2-[2-(n-dodecylamino)ethylamino]-ethylamine or the hydrochloride salt thereof in an amount of 0.005 to 0.10 percent based on the total weight of said teat dip.

7.  A teat dip concentrate or teat dip according to any one of claims 1 to 6, wherein a dye is included.

8.  A teat dip concentrate or teat dip according to any one of claims 1 to 7, wherein an antifoaming agent is included.

9.  A teat dip concentrate or teat dip according to any preceding claim wherein said emollient is a polyethylene glycol methyl glucoside.

10.  Use of a germicide for preparation of a medicament for controlling bovine mastitis in which the medicament is prepared by forming a teat dip concentrate or teat dip according to any preceding claim.

11.  Use as claimed in claim 10 wherein said emollient is a polyethylene glycol methyl glucoside.


**Patentansprüche**

1.  Keimtötendes Zitzenbademittelkonzentrat für Tiere, umfassend, als im wesentlichen stabile homogene Mischung, ein Germizid, das ausgewählt ist aus 3-(n-Dodecylamino)propylamin, einem pharmazeutisch verwendbaren Säureanlagerungssalz von 3-(n-Dodecylamino)propylamin, 2-[2-(n-Dodecylamino)-ethylamino]ethylamin, und einem pharmazeutisch verwendbaren Säureanlagerungssalz von 2-[2-(n-Dodecylamino)ethylamino]ethylamin, wobei das Germizid oder die Germizide in einer Gesamtmenge von 0,25 bis 2,5 Prozent basierend auf dem Gesamtgewicht des Zitzenbademittelkonzentrates vorhanden sind; als Emollientium ein polyethoxyliertes oder polypropoxyliertes Glucosid; als filmbildenden Bestandteil Poly(N-vinylpyrrolidon); und Wasser.

2.  Zitzenbademittelkonzentrat nach Anspruch 1, ferner umfassend N-[3-(n-Dodecylamino)propyl]glycin oder ein pharmazeutisch verwendbares Säureanlagerungssalz desselben.

3.  Zitzenbademittelkonzentrat nach einem der Ansprüche 1 und 2, dadurch gekennzeichnet, daß das Bademittelkonzentrat umfaßt: 3-(n-Dodecylamino)propylamin in einer Menge von 0,2 bis 1,8 Prozent basierend auf dem Gesamtgewicht des Zitzenbademittelkonzentrates,und 2-[2-(n-Dodecylamino)-ethylamino]ethylamin oder das Hydrogenchloridsalz desselben in einer Menge von 0,05 bis 0,7 Prozent basierend auf dem Gesamtgewicht des Zitzenbademittelkonzentrates.

4.  Zitzenbademittel aus einem Konzentrat nach Anspruch 1, umfassend eine germizid wirksame Menge des keimtötenden Mittels, wobei Wasser in einer Menge von mindestens etwa 90 Gew.-% vorhanden ist, basierend auf dem Gesamtgewicht des Zitzenbademittels.

5.  Zitzenbademittel nach Anspruch 4, ferner umfassend N-[3-(n-Dodecylamino)propyl]glycin oder ein pharmazeutisch verwendbares Säureanlagerungssalz desselben.

6.  Zitzenbademittel nach einem der Ansprüche 4 und 5, dadurch gekennzeichnet, daß das Zitzenbademittel enthält: 3-(n-Dodecylamino)propylamin oder das Hydrogenchloridsalz desselben in einer Menge von 0,025 bis 0,25 Prozent basierend auf dem Gesamtgewicht des Zitzenbademittels; und 2-[2-(n-Dodecylamino)ethylamino]ethylamin oder das Hydrogenchloridsalz desselben in einer Menge von 0,005 bis 0,10 Prozent basierend auf dem Gesamtgewicht des Zitzenbademittels.

7.  Zitzenbademittelkonzentrat oder Zitzenbademittel nach einem der Ansprüche 1 bis 6, dadurch gekennzeichnet, daß ein Farbstoff enthalten ist.

8.  Zitzenbademittelkonzentrat oder Zitzenbademittel nach einem der Ansprüche 1 bis 7, dadurch gekennzeichnet, daß ein Mittel gegen Schaumbildung enthalten ist.

9.  Zitzenbademittelkonzentrat oder Zitzenbademittel nach einem der vorhergehenden Ansprüche, dadurch gekennzeichnet, daß das Emollientium ein Polyethylenglycolmethylglucosid ist.

10. Verwendung eines keimtötenden Mittels für die Herstellung eines Medikaments zur Bekämpfung der Rindermastitis, wo das Medikament hergestellt wird mit Hilfe eines Zitzenbademittelkonzentrates oder Zitzenbademittels nach einem der vorhergehenden Ansprüche.

11. Verwendung nach Anspruch 10, dadurch gekennzeichnet, daß das Emollientium ein Polyethylenglycolmethylglucosid ist.

**Revendications**

1.  Concentré de composition germicide pour immerger les trayons des animaux, comprenant, sous forme de mélange homogène substantiellement stable, un germicide choisi parmi 3-(n-dodécylamino)-propylamine, un sel d'addition d'acides pharmaceutiquement acceptable de 3-(n-docécylamino)-propylamine, 2-[2-(n-dodécylamino)éthylamino]éthylamine, et un sel d'addition d'acides pharmaceutiquement acceptable de 2-[2-(n-dodécylamino)éthylamino]éthylamine, ledit germicide ou lesdits germicides étant contenu(s) en une quantité totale de 0,25 à 2,5 %, par rapport au poids total dudit concentré de composition pour immerger les trayons ; un glucoside polyéthoxylé ou polypropoxylé, comme émollient ; la poly(N-vinylpyrrolidone) comme agent filmogène ; et de l'eau.

2.  Concentré de composition pour immerger les trayons selon la revendication 1, contenant en outre la N-[3-(n-dodécylamino)propyl]glycine ou un sel d'addition d'acides de celle-ci, pharmaceutiquement acceptable.

3.  Concentré de composition pour immerger les trayons selon la revendication 1 ou 2, où ledit concentré de composition d'immersion contient la 3-(n-dodécylamino)propylamine en une quantité de 0,2 à 1,8 %, par rapport au poids total dudit concentré de composition d'immersion, et la 2-[2-(n-dodécylamino)-éthylamino]éthylamine, ou le sel chlorhydrate de celle-ci, en une quantité de 0,05 à 0,7 %, par rapport au poids total dudit concentré de composition d'immersion.

4.  Composition pour immerger les trayons, provenant d'un concentré selon la revendication 1, contenant une quantité efficace dudit germicide et au moins 90 % d'eau, par rapport au poids total de ladite composition pour immerger les trayons.

5.  Composition pour immerger les trayons selon la revendication 4, contenant en outre la N-[3-(n-dodécylamino)propyl]glycineou un sel d'addition d'acides de celle-ci, pharmaceutiquement acceptable.

6.  Composition pour immerger les trayons selon la revendication 4 ou 5, contenant 0,025 à 0,25 % de 3-(n-dodécylamino)propylamine ou du sel chlorhydrate de celle-ci, par rapport au poids total de ladite composition pour immerger les trayons, et 0,005 à 0,10 % de 2-[2-(n-dodécylamino)éthylamino]-éthylamine ou du sel chlorhydrate de celle-ci, par rapport au poids total de ladite composition pour immerger les trayons.

7.  Concentré de composition pour immerger les trayons ou composition pour immerger les trayons selon une quelconque des revendications 1 à 6, contenant un colorant.

8.  Concentré de composition pour immerger les trayons ou composition pour immerger les trayons selon une quelconque des revendications 1 à 7, contenant un agent anti-mousse.

9.  Concentré de composition pour immerger les trayons ou composition pour immerger les trayons selon une quelconque des revendications précédentes, où ledit émollient est un méthylglucoside de polyéthylèneglycol.

10. Utilisation d'un germicide pour la préparation d'un médicament pour maîtriser les mammites bovines, où on prépare le médicament en formant un concentré de composition pour immerger les trayons ou une composition pour immerger les trayons selon une quelconque des revendications précédentes.

**11.** Utilisation selon la revendication 10, où ledit émollient est un méthylglucoside de polyéthylèneglycol.